# EUROPEAN PATENT SPECIFICATION

(11) **EP 0 452 278 B1**
(45) Date of publication and mention of the grant of the patent: **02.11.1995**
(21) Application number: 91830116.9
(22) Date of filing: 26.03.1991
(51) Int. Cl.: A61N 1/05

(54) **A positively anchored retractable tripolar catheter for endocardial pacemaker electrodes**
Mit positiver Verankerung, zurückziehbarer, dreipoliger Katheter für endokardische Herzschrittmacherelektroden
Cathéter tripolaire rétractable à verrouillage positif pour électrodes endocardiaques de stimulateur cardiaque

(30) Priority: 05.04.1990 IT 343390
(43) Date of publication of application: 16.10.1991
(73) Proprietor: X-TRODE S.r.l., 40129 Bologna (IT)
(72) Inventor: Borghi, Enzo, I-40054 Budrio (Bologna) (IT); Antonioli, Gian Enrico, I-44100 Ferrara (IT)
(74) Representative: Lanzoni, Luciano

(56) References cited:
- WO-A-83/04181
- FR-A- 2 493 153
- GB-A- 2 116 047

## Description

The present invention relates to a retractable tripolar electrode catheter for use in conjunction with artificial cardiac pacemakers, of the type that can be anchored positively to the endocardium. The art field of heart surgery embraces numerous types of cardiac catheter designed for connection at one end, generally by way of a flexible tube functioning as a biocompatible outer sheath, to an artificial pacemaker implanted in the body of the patient, and carrying a terminal or distal electrode at the other end which can contact (or can be anchored positively to) the ventricular cardiac muscle.

The typical catheter also incorporates a second (proximal) electrode distanced and insulated from the first, which is positioned so as to occupy the atrium once the terminal electrode is anchored.

The terminal, or ventricular electrode, consists generally in a helicoid point that is rotated by hand in such a way as to corkscrew into and thus establish a continuous and secure contact with the cardiac muscle; this point is connected to a first terminal of the pacemaker by way of an electrically conductive spiral-wound wire. The atrial electrode consists in a ring (or a set of rings spaced apart one from the next) fashioned in biocompatible metal and encircling the outer sheath of the catheter. These rings provide the atrial contact, and are connected to a second terminal of the pacemaker by way of a single spiral-wound conductor coaxial with and encompassing the first; the two conductors are isolated from one another electrically by means of a second tubular insulator ensheathing the first conductor: this realizes a so called bipolar lead.

According to conventional surgery techniques, the catheter is inserted, or manoeuvred, in such a way as to locate the atrial ring in direct contact with the lining of the heart, whereas in more recent methods the atrial signal is sensed directly from the bloodstream.

In the latter instance, the atrial electrode acts as a floating means of monitoring haematic flow in such a way as to return a suitable atrial signal to the sensing circuit of the pacemaker device; having analyzed the signal, the pacemaker produces the appropriate stimulation response via its internal components on the basis of the signal received.

Thus, the facility of obtaining greater precision in control of haematic flow and consequently a more precise atrial signal results ideally in optimized artificial cardiac stimulation.

Recently, surgeons have attempted to introduce several bipolar leads into the heart of a patient in order to better accomplish atrio-ventricular AV sequential pacing. Ordinarily, the veins are surgically opened and endocardials leads are introduced through the respective venous openings. The desirability of inserting two endocardial leads to achieve AV sequential pacing has posed a problem to the surgeon, since two - rather than only one - incisions must be made in two veins. Further when multiple leads are lodged in different portions of the heart the leads sometimes rub against each other due to lead flexing caused by beating of the heart. Accordingly, in the pubblication WO-A-83/04181, is provided a system and method of intravenously introducing an endocardial lead into a patients heart with minimum danger of dislodging an endocardial lead previously introduced and lodged within the patient's heart; the leads being of bipolar type as descripted above with a distal ventricular electrode and a proximal atrial electrode.

Accordingly, the notion occurs to the applicant of designing a positively anchored type of electrode catheter that permits of more accurate monitoring, hence the acquisition of an optimized, and as far as possible a "clean" atrial signal derived from haematic flow, with a drastic reduction in those variable eternal factors that could influence the reception of the signal. The document GB-A-2 116 047 discloses a tripolar catheter with spiral wound conductors and two atrial electrodes consisting of metal rings. The ventricular conductor is monopolar and slidable in the longitudinal direction of the catheter.

The object of the present invention is to overcome the drawbacks betrayed by conventional electrodes through the provision of a tripolar and positively anchored electrode catheter which, by virtue of the special structure of the atrial electrode, permits of monitoring atrial haematic flow in real time, comparatively, swiftly and accurately, and using a catheter of compact dimensions.

The stated object is realized in a cardiac catheter according to the present invention, which comprises a flexible tubular casing embodied in biocompatible material, fitted with a ventricular electrode at the leading end and ensheathing three distinct electrically conductive spiral-wound wires disposed parallel one with another: a first conductor, of which one end is connected by way of a relative first contact to the first terminal of a cardiac pacemaker and the remaining end to a helicoid point invested with movement by rotating the spiral-wound wire from a position retracted internally of the tubular casing to a position projecting from the casing in such a way as to embed and anchor the point positively in the ventricular heart muscle; a second conductor of which one end is connected by way of a second contact to a second terminal of the pacemaker and the remaining end to a first bio-compatible metal ring positioned externally of the casing and at a predetermined distance from the point in such a way as to provide a first atrial contact; and a third conductor of which one end is connected by way of a third contact to a third terminal of the pacemaker and the remaining end to a second biocompatible metal ring, similar to the first and distanced therefrom in such a way as to provide a second atrial contact.

The first contact is separated from the casing in such a way as to enable a connection between the electrodes and the pacemaker by way of two distinct and substantially parallel connector leads of which the first is unipolar and the second bipolar.

One of the advantages of the present invention is that it provides a dual atrial contact, consisting in two metal rings distanced one from the other, by means of which haematic flow can be monitored with a greater degree of precision (the rings do not make contact one with the other) and a more precise atrial signal thus returned to the sensing circuit of an artificial pacemaker.

The invention will now be described in detail, by way of example, with the aid of the accompanying drawings, in which:
- fig 1 illustrates the electrode catheter according to the present invention in a side elevation;
- fig 2 is the enlarged side elevation of a forked end part of the catheter of fig 1 seen with certain parts omitted better to reveal others;
- fig 3 is the section through III-III, fig 2;
- fig 4 is a side elevation of the part of the catheter fitted with atrial contact rings, viewed with certain parts omitted better to reveal others;
- fig 5 is the section through V-V, fig 4;
- fig 6 is the section through VI-VI, fig 2.

As illustrated in the accompanying drawings, a retractable cardiac electrode catheter according to the present invention comprises a flexible tubular casing 1 fashioned from biocompatible material in such a way as to form a longitudinally insertable element, furnished at the inserted extremity with a ventricular electrode 2 such as can be introduced into the cavity of the heart and used to anchor the catheter positively to the cardiac muscle.

The casing 1 contains three distinct spiral-wound conductors 3, 7 and 11 respectively (see figs 2 to 5); the first, denoted 3, embodied in electrically conductive wire and encompassed by a first tubular sheath of electrically insulating flexible material denoted 4, is rotatable within and axially slidable in relation to the casing 1, and connected at one end by way of a first contact 5 (conventional in embodiment and therefore not illustrated in full), to the first terminal of an implanted cardiac stimulation device (a conventional pacemaker, not illustrated).

The remaining end of the conductor 3 is fastened, for example by a conventional crimping or clinching process, to a helicoid point 6 that constitutes the ventricular electrode 2.

The point 6 is capable of movement, produced by rotation of the spiral-wound conductor 3, from a retracted position internally of the casing 1 to a position extended from the casing in which it can be anchored to the lining of the ventricle.

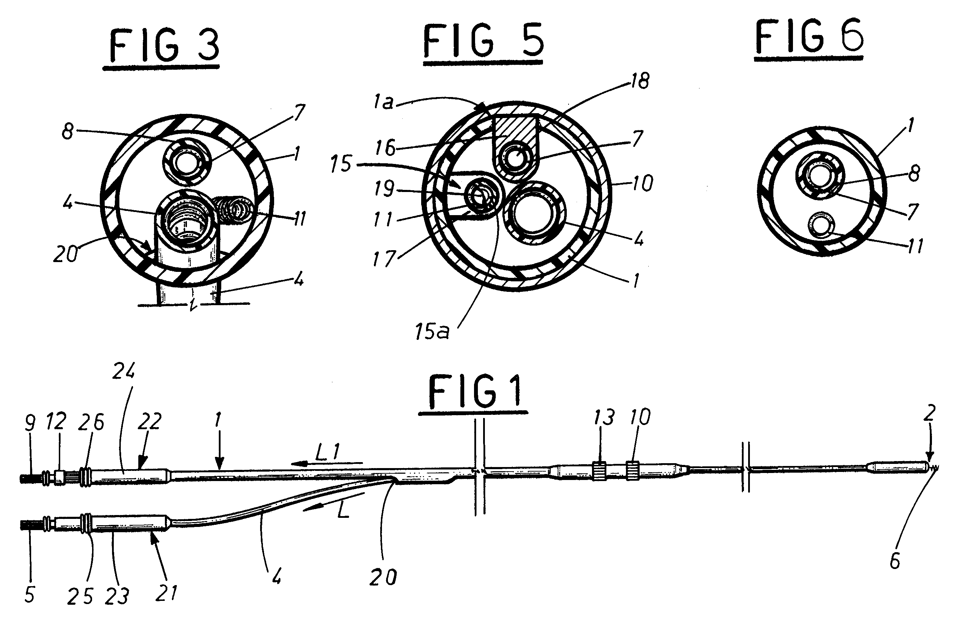

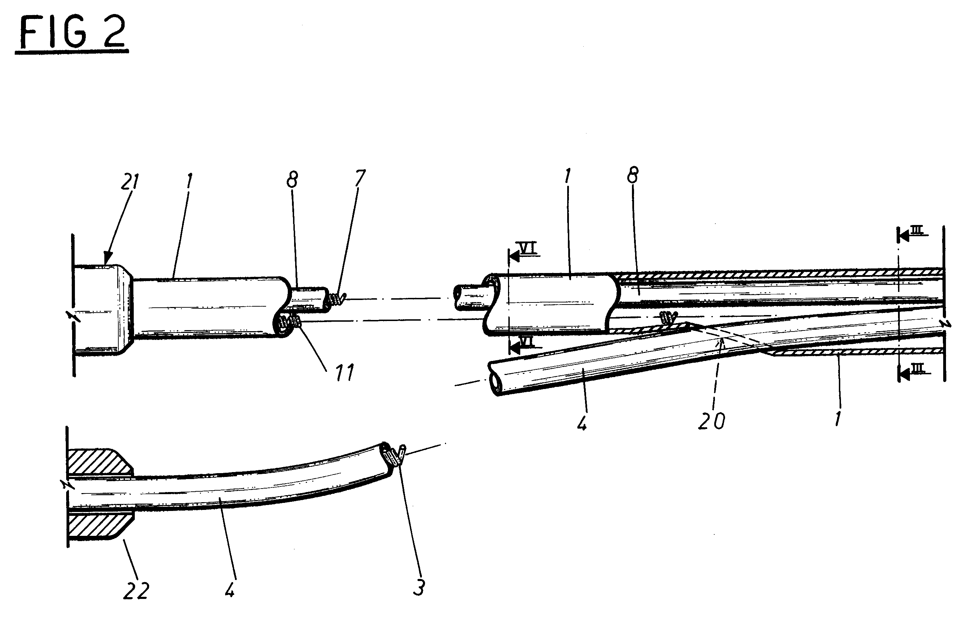

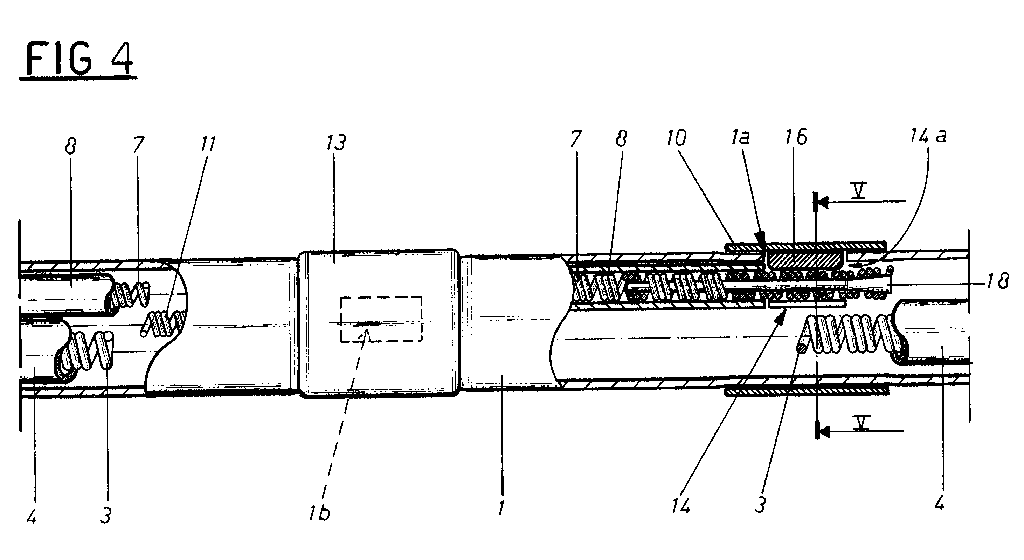

The second conductor 7, a spiral-wound electrically conductive wire encompassed in like manner to the first conductor 3 by a second sheath 8 similar in all respects to the first sheath 4, and disposed parallel to the first conductor 3 internally of the tubular casing 1, is connected at one end, by way of a second contact 9, to a second terminal of the pacemaker; the remaining end of the conductor 7 is connected to a first ring 10 fashioned from a metal of biocompatible specifications. This first ring 10 (to be described fully in due course), is located externally of the casing 1 (see figs 1 and 4) at a prescribed distance from the helicoid point 6 in such a way as to establish a first atrial contact. The third spiral-wound conductor 11 accommodated by the casing 1, disposed parallel to the first and the second and embodied likewise in electrically conductive wire, is connected at the one end to a third terminal of the pacemaker, by way of a third contact 12, and at the remaining end to a second ring 13 of biocompatible material; this second ring is located externally of the casing 1, isolated and distanced from the first ring 10 (in the example of fig 1, at a greater distance from the ventricular electrode 2), in such a way as to create a second atrial contact.

As illustrated in figs 1 and 2, the first contact 5 (i.e. the ventricular) is separated from the main body of the casing 1 in such a way as to allow of connecting the electrodes to the pacemaker by way of two distinct and substantially parallel leads L and L1 of which the first, serving the ventricular electrode, is unipolar or single core, and the second, serving the atrial electrodes, is bipolar or twin core.

More exactly, the first contact 5 is joined by way of a one-pin connector 21 to the first sheath 4, which emerges from the casing 1 at a position near to the end farthest from the point 6 to provide the one lead L, and the second contact 9 by way of a two-pin connector 22 to the casing 1, of which the corresponding part provides the remaining lead L1; the connectors 21 and 22 consist each in a metal plug 23 and 24 provided with corresponding stop collars 25 and 26 (see fig 1).

The first sheath 4 emerges from the casing 1 by way of a longitudinal slot 20 occupying a relative part of the cylindrical surface of the casing (clearly illustrated in figs 2 and 3).

The first and second atrial electrode rings 10 and 13 are associated with respective first and second couplers 14 and 15 disposed radially and sealed internally of the casing 1; each such coupler 14 and 15 affords a corresponding socket 14a and 15a, radially offset one from the other and from the first spiral-wound conductor 3 (fig 5), by way of which electrical contact is established between the atrial electrodes 10 and 13 and their respective conductors 7 and 11.

Observing figs 4 and 5, each of the couplers 14 and 15 associated with the first and second rings 10 and 13 will be seen to comprise a sleeve 16 and 17 associated radially with the internal circumference of the respective first and second ring 10 and 13 and insertable into the corresponding part of the casing 1 by way of and in sealed association with a longitudinal slot 1a and 1b. Each sleeve 16 and 17 accommodates a respective coaxial pin 18 and 19 fashioned in electrically conductive material, over which the end of the corresponding spiral-wound conductor 7 and 11 is coaxially and stably secured. The remaining ends of these two conductors 7 and 11 converge into the relative pacemaker connector 22 (see also fig 6) and combine with the casing 1 and the respective second and third contacts 9 and 12 to provide the twin core lead L1.

A cardiac catheter embodied in this manner affords notable advantages in comparison to conventional types, first among which is that the ventricular electrode remains electrically and mechanically independent from the remainder of the catheter and can be anchored to the lining of the heart easily and precisely (several times if necessary) without manipulating the remaining parts of the casing; the relative spiral-wound conductor 3 can be of minimal diametral dimensions moreover (a factor reflecting favourably on the overall dimensions of a tripolar embodiment) without prejudice to its mechanical strength and manoeuvrability.

The second and third spiral-wound conductors effect a dual atrial control by monitoring haematic flow at two locations, independent of and remote from one another, thereby returning two signals to the sensing circuit of the pacemaker device (which will incorporate independent data circuits) for analysis and comparison, and enabling the application of a more precise and correctly attuned pulse to the ventricle by way of the relative electrode 6 in response.

Likewise to advantage, the provision of atrial contact rings with radially offset coupling sleeves permits of positioning the conductors parallel with one another and thus establishing three independent circuits (without notably affecting the dimensions of the casing); with a significant reduction in the possibility of mutual interference during passage of the respective pulses moreover, input and output signals to and from the pacemaker are guaranteed essentially "clean". In addition, the particular embodiment of the rings and the inclusion of the longitudinal slot 20 combine to ensure an outer casing 1 of unbroken continuity, with all of the attendant advantages in terms of strength, sealing action, manoeuvrability and long term reliability.

## Claims

1. A positively anchorable retractable tripolar catheter for endocardial pacemaker electrodes,
which comprises a flexible tubular casing (1) of biocompatible material fitted with a ventricular electrode (2) at the leading end and ensheathing:
- a first conductor (3) of spiral-wound electrically conductive wire accommodated within a first tubular sheath (4) fashioned in a flexible and electrically insulating material, internally of which the spiral wound wire is rotatable and longitudinally slidable within and in relation to the casing (1), of which one end is connectable by way of a first contact (5) to a first terminal of a cardiac pacemaker and the remaining end is fastened to an electrically conductive helicoid point (6) capable of movement, produced by rotation of the first conductor (3), from a position retracted internally of the casing to a position projecting from the casing, in such a way as to anchor in a ventricular cardiac muscle;
- a second spiral-wound conductor (7) accommodated within a second tubular sheath (8) similar to the first sheath (4) and disposed parallel with the first conductor (3) internally of the casing (1), of which one end is connectable by way of a second contact (9) to a second terminal of the pacemaker and the remaining end is connected to a first atrial electrode consisting of a first ring (10) of biocompatible metal positioned externally of the casing (1) at a predetermined distance from the point (6);
- a third spiral-wound conductor (11) disposed parallel with the first and the second conductors internally of the casing (1), of which one end is connectable by way of a third contact (12) to a third terminal of the pacemaker and the remaining end is connected to a second atrial electrode consisting of a second biocompatible metal ring (13) positioned externally of the casing (1) at a given distance from the first ring (10); and
in that the first contact (5) is separated from the casing (1) in order to permit connection of the electrodes and the pacemaker by way of two distinct and substantially parallel leads (L, L1), the one unipolar and the other bipolar.

2. An electrode catheter according to claim 1, characterized in that said first ring (10) is associated with a first coupler (14) that is radially disposed and sealed within the casing (1) and affords a corresponding socket (14a), radially offset from the first spiral-wound conductor (3), in which to accomodate the end of the second spiral-wound conductor (7);
and in that said second ring (13) is associated with a second coupler (15) radially disposed and sealed within the casing and affording a corresponding socket (15), radially offset from the first and second spiral-wound conductors (3, 7), in which to accomodate the end of the third spiral-wound conductor (11).

3. An electrode catheter as in claim 2, wherein the first coupler (14) and the second coupler (15) consist in respective sleeves (16, 17) disposed radially and within the circumference of the first and second rings (10, 13) and insertable into the casing (1) by way of and in sealed association with corresponding longitudinal slots (1a, 1b), and in respective electrically conductive pins (18, 19) accommodated coaxially by the sleeves (16, 17), over which the corresponding ends of the second and third conductors (7, 11) are coaxially and stably secured.

4. An electrode catheter as in claim 1, wherein the unipolar lead (L) coincides with a part of the first sheath (4) that emerges from the casing (1) near to the connection with the pacemaker by way of a longitudinal slot (20) formed in the cylindrical surface of the casing and is joined to the first contact (5) by way of a connector (21) associated with the pacemaker.

5. An electrode catheter as in claim 1, wherein the bipolar connecting lead (L1) coincides with parts of the second and third conductors (7, 11) that converge internally of the casing (1) toward substantially coaxial second and third contacts (9, 12) and are joined to a single connector (22) associated with the pacemaker.

## Patentansprüche

1. Ein positiv verankerter, zurückziehbarer, dreipoliger Katheter für endokardische Herzschrittmacherelektroden, enthaltend eine flexible schlauchförmige Umhüllung (1) aus biokompatiblem Material, welche mit seinem leitenden Ende an eine Herzkammerelektrode (2) angeschlossen ist und wie folgt enthält:
- Einen ersten Leiter (3), bestehend aus einem elektrisch leitenden, spiralförmig gewundenen Draht, der in eine erste schlauchförmige Hülle (4) eingebettet ist, bestehend aus einem flexiblen, elektrisch isolierenden Material, und in deren Innerem der spiralförmig gewundene Draht im Verhältnis zu der Umhüllung (1) drehbar und in Längsrichtung gleitbar ist, und dessen eines Ende über einen ersten Kontakt (5) an einen ersten Pol eines Herzschrittmachers angeschlossen ist, und dessen verbleibendes Ende an eine elektrisch leitende schraubenförmige Spitze (6) angeschlossen ist, die in der Lage ist, hervorgerufen durch die Umdrehung des ersten Leiters (3), eine Bewegung aus einer in das Innere der Umhüllung zurückgezogenen Position in eine aus der Umhüllung hervorstehende Position auszuführen, und zwar auf solche Weise, dass sie in einem Herzkammermuskel verankert wird;
- einen zweiten spiralförmig gewundenen Leiter (7), eingebettet in einer zweite schlauchförmige Hülle (8) ähnlich wie die erste Hülle (4) und angeordnet parallel zu dem ersten Leiter (3) im Inneren der Umhüllung (1), dessen eines Ende über einen zweiten Kontakt (9) an einen zweiten Pol des Herzschrittmachers anschliessbar ist, und dessen verbleibendes Ende an eine erste Vorkammerelektrode angeschlossen ist, bestehend aus einem ersten Ring (10) aus biokompatiblen Metall und angeordnet ausserhalb der Umhüllung (1) in einem bestimmten Abstand von der Spitze (6);
- einen dritten spiralförmig gewundenen Leiter (11), angeordnet parallel zu den ersten und zweiten Leitern im Inneren der Umhüllung (1), dessen eines Ende über einen dritten Kontakt (12) an einen dritten Pol des Herzschrittmachers anschliessbar ist, und dessen verbleibendes Ende an eine zweite Vorkammerelektrode angeschlossen ist, bestehend aus einem zweiten Ring (13) aus biokompatiblen Metall und angeordnet ausserhalb der Umhüllung (1) in einem bestimmten Abstand von dem ersten Ring (10);
wobei der erste Kontakt (5) von der Umhüllung (1) getrennt ist, so dass die Verbindung der Elektroden und des Herzschrittmachers über zwei unterschiedliche und im wesentlichen parallele Leitungen (L, L1) erfolgt, von denen die eine einpolig und die andere zweipolig ist.

2. Elektrodenkatheter nach Patentanspruch 1, **dadurch gekennzeichnet**, dass der genannte erste Ring (10) einem ersten Verbinder (14) zugeordnet ist, radial und dicht abschliessend im Inneren der Umhüllung (1) angeordnet und einen entsprechenden Sitz (14a) aufweisend, der gegenüber dem ersten spiralförmig gewundenen Leiter (3) radial versetzt ist, und in dem das Ende des zweiten spiralförmig gewundenen Leiters (7) aufgenommen wird; **und dadurch**, dass der genannte zweite Ring (13) einem zweiten Verbinder (15) zugeordnet ist, radial und dicht abschliessend im Inneren der Umhüllung angeordnet und einen entsprechenden Sitz (15a) aufweisend, der gegenüber den ersten und zweiten spiralförmig gewundenen Leitern (3, 7) versetzt ist, und in dem das Ende des dritten spiralförmig gewundenen Leiters (11) aufgenommen wird.

3. Elektrodenkatheter nach Patentanspruch 2, **dadurch gekennzeichnet**, dass der erste Verbinder (14) und der zweite Verbinder (15) aus jeweiligen Buchsen (16, 17) bestehen, die radial und innerhalb des Umfangs der genannten ersten und zweiten Ringe (10, 13) angeordnet und in die Umhüllung (1) einsetzbar sind, und zwar durch und in abdichtender Verbindung mit entsprechenden Längsschlitzen (1a, 1b) und mit jeweiligen elektrisch leitenden, koaxial von den Buchsen (16, 17) aufgenommenen Stiften (18, 19), auf welche die entsprechenden Enden der zweiten und dritten Leiter (7, 11) koaxial und stabil aufgezogen sind.

4. Elektrodenkatheter nach Patentanspruch 1, **dadurch gekennzeichnet**, dass die einpolige Leitung (L) mit einem Teil der ersten Hülle (4) übereinstimmt, der dicht an der Verbindungsstelle mit dem Herzschrittmacher aus der Umhüllung (1) herausragt, und zwar durch einen Längsschlitz (20), der in die zylindrische Oberfläche der Umhüllung eingearbeitet ist, und der an den ersten Kontakt (5) über einen dem Herzschrittmacher zugeordneten Verbinder (21) angeschlossen ist.

5. Elektrodenkatheter nach Patentanspruch 1, **dadurch gekennzeichnet**, dass die zweipolige Verbindungsleitung (L1) mit Teilen der zweiten und dritten Leiter (7, 11) übereinstimmt, die im Inneren der Umhüllung (1) zu im wesentlichen koaxialen zweiten und dritten Kontakten (9, 12) konvergieren und an einen einzigen Verbinder (22) angeschlossen sind, der dem Herzschrittmacher zugeordnet ist.

## Revendications

1. Un cathéter tripolaire rétractable à verrouillage positif pour électrodes endocardiaques de stimulateur cardiaque, comprenant un logement souple tubulaire (1) en matière biocompatible dotée à son extrémité d'une électrode ventriculaire (2) et enveloppant:
- un premier conducteur (3) de fil conducteur électrique enroulé en spirale logé à l'intérieur d'une première gaine tubulaire (4) réalisé dans un matériau flexible et électriquement isolant, à l'intérieur de laquelle le fil enroulé en spirale peut tourner et coulisser longitudinalement à l'intérieur et par rapport au logement (1), d'une lune des extrémités est relié au moyen d'un premier contact (5) au premier pôle d'un simulateur cardiaque et l'autre extrémité est fixée à un élément hélicoïde électriquement conducteur (6), pouvant se déplacer par la rotation du premier conducteur (3) d'une position rétractée à l'intérieur du logement à une position extérieure au logement, de façon à se verrouiller dans un muscle cardiaque ventriculaire;
- un deuxième conducteur en spirale (7) logé à l'intérieur d'une deuxième gaine tubulaire (8) similaire à la première gaine (4) et disposé parallèlement au premier conducteur (3) à l'intérieur du logement (1), dont l'une des extrémités peut être reliée au moyen d'un deuxième contact (9) au deuxième pôle du stimulateur cardiaque et l'autre extrémité est relié à la première électrode atrio-ventriculaire consistant en un premier anneau (10) de métal biocompatible situé à l'extérieur du logement (1) à une distance donnée de l'élément (6);
- un troisième conducteur en spirale (11) disposé parallèlement au premier et au deuxième conducteur à l'intérieur du logement (1), dont une extrémité peut être reliée au moyen d'un troisième contact (12) à un troisième pôle du stimulateur cardiaque et l'autre extrémité est reliée à une deuxième électrode atrioventriculaire consistant en un deuxième anneau en métal biocompatible (13) situé à l'extérieur du logement (1), à une distance donnée du premier anneau (10);
le premier contact (5) est séparé du logement (1) de façon à permettre le raccordement des électrodes et du stimulateur cardiaque au moyen de deux fils distincts et approximativement parallèles (L, L1), l'un unipolaire et l'autre bipolaire.

2. Un cathéter à électrodes selon la revendication 1, caractérisé en ce que ledit premier anneau (10) est associé à un premier coupleur (14), lequel est disposé radialement et soudé à l'intérieur du logement (1) et présente une prise (14a), radiale par rapport au premier conducteur en spirale (3), dans laquelle il est possible de ficher l'extrémité du deuxième conducteur en spirale (7);
et en ce que ledit deuxième anneau (13) est associé à un deuxième coupleur (15) lequel est disposé radialement et soudé à l'intérieur du logement (1) et présente une prise (15a) radiale par rapport au premier et au deuxième conducteur en spirale (3, 7), dans laquelle il est possible de ficher l'extrémité du troisième conducteur en spirale (11);

3. Un cathéter à électrodes selon la revendication 2, dans lequel le premier coupleur (14) et le deuxième coupleur (15) consistent en des manchons (16, 17) disposés radialement et à l'intérieur de la circonférence du premier et du deuxième anneau (10, 13) et pouvant être introduits à l'intérieur du logement (1) au moyen de et en association soudée avec les fentes longitudinales correspondantes (1a, 1b), et dans des fiches électriquement conductrices (18,19) disposées coaxialement par les manchons (16, 17), au-dessus desquelles les extrémités correspondantes du deuxième et du troisième conducteurs (7, 11) sont coaxialement et solidement assurées.

4. Un cathéter à électrodes selon la revendication 1, dans lequel le fil unipolaire (L) coïncide avec une partie de la première gaine (4) qui émerge du logement (1) près de la connexion avec le stimulateur cardiaque par une fente longitudinale (20) pratiquée dans la surface cylindrique du logement, et est joint au premier contact (5) au moyen du conducteur (21) associé au stimulateur cardiaque.

5. Un cathéter à électrodes selon la revendication 1, dans lequel le fil bipolaire (L1) coïncide avec des parties du deuxième et du troisième conducteurs (7, 11) qui convergent à l'intérieur du logement (1) vers le deuxième et le troisième contacts (9, 12) substantiellement coaxiaux et sont joints à un unique connecteur (22) associé au stimulateur cardiaque.
